# FASCICULE DE BREVET EUROPEEN

(11) **EP 0 577 473 B1**
(45) Date de publication et mention de la délivrance du brevet: **20.12.1995**
(21) Numéro de dépôt: 93401615.5
(22) Date de dépôt: 23.06.1993
(51) Int. Cl.: A61K 7/09, A61K 7/06

(54) **Composition cosmétique réductrice pour la déformation permanente des cheveux à base d'un mercaptoalkylamino-amide et procédé de déformation permanente des cheveux**
Reduzierende kosmetische Zusammensetzung zur Haardauerwellung auf Basis eines Mercaptoalkylamino-Amids und Haardauerwellverfahren
Reducing cosmetic composition for the permanent deformation of hair based on a mercaptoalkylamino-amide and process for the permanent deformation of hair

(30) Priorité: 23.06.1992 FR 9207641
(43) Date de publication de la demande: 05.01.1994
(73) Titulaire: L'OREAL, 75008 Paris (FR)
(72) Inventeur: Malle, Gérard, F-77580 Villiers Sur Morin (FR); Luppi, Bernadette, F-93270 Sevran (FR)
(74) Mandataire: Stalla-Bourdillon, Bernard

(56) Documents cités:
- EP-A- 0 432 000
- DE-A- 4 026 541
- FR-A- 2 668 929
- US-A- 3 093 146
- JOURNAL OF ORGANIC CHEMISTRY vol. 32, no. 7, Juillet 1967, EASTON US pages 2344 - 2346 JOHNSTON T. P. ET AL 'N-(2-Mercaptoethyl)alanine and related compounds'

## Description

La présente invention a pour objet une composition cosmétique réductrice, pour le premier temps d'une opération de déformation permanente des cheveux, contenant en tant qu'agent réducteur un mercaptoalkylamino-amide ou l'un des ses sels cosmétiquement acceptables, et son utilisation dans un procédé de déformation permanente des cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou leur décrêpage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un thiol.

Parmi ces derniers, ceux couramment utilisés sont la cystéine et l'acide thioglycolique ainsi que ses esters, notamment le monothioglycolate de glycérol.

L'acide thioglycolique est particulièrement efficace pour réduire les liaisons disulfures de la kératine et peut être considéré à pH alcalin, notamment sous forme de thioglycolate d'ammonium, comme le composé de référence en permanente. Il présente cependant l'inconvénient de dégager une odeur désagréable. En vue d'y remédier, il est généralement fait usage d'un parfum permettant de masquer les odeurs.

La cystéine produit une odeur beaucoup plus faible que celle de l'acide thioglycolique mais le degré de frisure obtenu est très inférieur et loin d'être satisfaisant. De plus, la cystéine nécessite l'utilisation d'un pH très alcalin.

Le monothioglycolate de glycérol est également très malodorant. Il est par contre utilisé à un pH proche de la neutralité mais ses performances sont notablement inférieures à celles de l'acide thioglycolique.

Diverses études ont été conduites en vue de remédier aux inconvénients de ces agents réducteurs, et à cet effet il a été proposé l'emploi de nouveaux composés réducteurs. Ainsi, dans la demande de brevet japonais n° 138113-1990, il a été décrit l'utilisation de la N-carboxyméthyl cystéamine (HS-CH₂-CH₂-NH-CH₂-COOH).

Après diverses études, on a constaté de façon tout à fait surprenante et inattendue, qu'à molarité équivalente, l'amide primaire de la N-carboxyméthyl cystéamine ou (mercapto-2-éthylamino)-2 acétamide permettait d'obtenir un rendement de frisure très supérieur à celui obtenu à l'aide de la N-carboxyméthyl cystéamine et à celui obtenu, selon l'état de la technique, à l'aide de l'acide thioglycolique. La poursuite de ces études a permis de mettre en évidence de façon tout à fait inattendue que cette observation était générale aux amides d'amino-acides dont la fonction amine est substituée par le reste mercapto-2 éthyle. Ces composés présentent un effet des performances de frisure tout à fait exceptionnelles tout en possédant une odeur très réduite. Toutefois, il est bien connu que plus le rendement de frisure est élevé, plus les cheveux sont abîmés. Or on a constaté de façon tout à fait surprenante qu'avec ces nouveaux composés, l'amélioration de la frisure était obtenue sans détérioration de l'état des cheveux. Les agents réducteurs des compositions selon l'invention permettent par ailleurs d'obtenir une nervosité et une beauté de frisure supérieures à celles obtenues selon l'état de la technique.

La présente invention a donc pour objet, à titre de produit industriel nouveau, une composition cosmétique pour le premier temps d'une opération de déformation permanente des cheveux contenant, dans un véhicule cosmétique approprié, en tant qu'agent réducteur, au moins un mercaptoalkylamino-amide répondant à la formule générale suivante :

HS-CH₂-CH₂-NH-A-CO-NR₁R₂ (I)

dans laquelle :
A représente le radical divalent (̵CH₂)ₙ- , n étant un nombre entier compris entre 1 et 5,
ou le radical 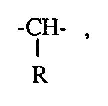
R représentant un radical alkyle inférieur, linéaire ou ramifié, ayant de 1 à 5 atomes de carbone ou le radical méthylthio-2 éthyle,
R₁ et R₂, identiques ou différents, représentent un atome d'hydrogène ou un radical alkyle inférieur, linéaire ou ramifié, ayant de 1 à 3 atomes de carbone ou un radical hydroxyalkyle ayant de 1 à 4 atomes de carbone,
ou un sel desdits composés de formule (I).

Parmi les sels cosmétiquement acceptables des composés de formule (I), ceux particulièrement préférés sont les chlorhydrates, bromhydrates, citrates, oxalates et acétates.

Par radical alkyle inférieur, linéaire ou ramifié, ayant de 1 à 5 ou de 1 à 3 atomes de carbone, on doit entendre un radical méthyle, éthyle, isopropyle, isobutyle ou méthyl-1 propyle.

Par radical hydroxyalkyle ayant de 1 à 4 atomes de carbone, on doit entendre un radical hydroxy-2 éthyle, hydroxy-2 propyle, hydroxy-3 propyle ou hydroxyméthyl-1 propyle.

Parmi les composés préférés correspondant à la formule générale (I), on peut notamment citer :
- le (mercapto-2 éthylamino)-2 acétamide,
- le (mercapto-2 éthylamino)-2 propionamide,
- le (mercapto-2 éthylamino)-3 propionamide,
- le (mercapto-2 éthylamino)-2 méthylthio-4 butanamide,
- le (mercapto-2 éthylamino)-2 méthyl-3 butanamide,
- le (mercapto-2 éthylamino)-2 méthyl-4 pentanamide,
- le (mercapto-2 éthylamino)-2 méthyl-3 pentanamide,
- le (mercapto-2 éthylamino)-2 N-méthyl acétamide,
- le (mercapto-2 éthylamino)-2 N-éthyl acétamide,
- le (mercapto-2 éthylamino)-2 N-propyl acétamide,
- le (mercapto-2 éthylamino)-2 N-isopropyl acétamide,
- le (mercapto-2 éthylamino)-2 N,N-diméthyl acétamide,
- le (mercapto-2 éthylamino)-2 N,N-diéthyl acétamide,
- le (mercapto-2 éthylamino)-2 N-(hydroxy-2' éthyl)acétamide,
- le (mercapto-2 éthylamino)-2 N,N-di(hydroxy-2' éthyl)acétamide,
- le (mercapto-2 éthylamino)-2 N-méthyl propionamide,
- le (mercapto-2 éthylamino)-2 N,N-diméthyl propionamide,
- le (mercapto-2 éthylamino)-2 N-éthyl propionamide,
- le (mercapto-2 éthylamino)-2 N-(hydroxy-2' éthyl)propionamide,
- le (mercapto-2 éthylamino)-2 N,N-di(hydroxy-2' éthyl)propionamide,
- le (mercapto-2 éthylamino)-3 N-méthyl propionamide,
- le (mercapto-2 éthylamino)-3 N-(hydroxy-2' éthyl) propionamide,
- le (mercapto-2 éthylamino)-2 méthylthio-4 N-méthyl butanamide et
- le (mercapto-2 éthylamino)-2 méthylthio-4 N-(hydroxy-2' éthyl)butanamide,
ainsi que leurs chlorhydrates.

Les composés de formule générale (I) sont pour certains nouveaux et leur procédé de préparation sera décrit plus en détail ci-après.

Dans les compositions de permanente selon l'invention, l'agent réducteur de formule générale (I) est généralement présent à une concentration comprise entre 1 et 30 % et de préférence entre 5 et 20% en poids par rapport au poids total de la composition réductrice.

Le pH de la composition est de préférence compris entre 4 et 11 et plus particulièrement entre 6 et 10 et est obtenu à l'aide d'un agent alcalin tel que par exemple l'ammoniaque, la monoéthanolamine, la diéthanolamine, la triéthanolamine, la propanediamine-1,3, un carbonate ou bicarbonate alcalin ou d'ammonium, un carbonate organique tel que le carbonate de guanidine, un hydroxyde alcalin ou à l'aide d'un agent acidifiant tel que par exemple l'acide chlorhydrique, l'acide acétique, l'acide lactique, l'acide oxalique ou l'acide borique.

La composition réductrice peut également contenir en association un autre agent réducteur connu tel que par exemple l'acide thioglycolique, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide β-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terteux, les N-(mercaptoalkyl)ω-hydroxyalkylamides décrits dans la demande de brevet EP 354 835 et les N-mono- ou N,N-dialkylmercapto 4-butyramides décrits dans la demande de brevet EP 368763, les aminomercaptoalkylamides décrits dans la demande de brevet EP 403 267 et les alkylaminomercaptoalkylamides décrits dans la demande de brevet EP 432 000.

Selon un mode de réalisation préféré, la composition réductrice contient également un agent tensioactif de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, mais de préférence comprise entre 0,5 et 10 % en poids par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevets français n° 2.598.613 et n° 2.470.596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n° 4.749.732, des polyorganosiloxanes tels que le copolymère polydiméthylsiloxanepolyoxyalkyle du type Diméthicone Copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

La composition réductrice peut également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n°83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

La composition réductrice selon l'invention se présente essentiellement sous forme aqueuse notamment sous la forme d'une lotion épaissie ou non, d'une crème ou d'un gel.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

La composition réductrice selon l'invention peut également contenir un solvant tel que par exemple de l'éthanol, du propanol, ou de l'isopropanol ou encore du glycérol à une concentration maximale de 20 % par rapport au poids total de la composition.

Le véhicule des compositions selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.....

On peut également utiliser des liquides ou des gels contenant des agents épaississants tels que des polymères ou des copolymères carboxyvinyliques qui "collent" les cheveux et les maintiennent dans la position lisse pendant le temps de pose.

Les compositions selon l'invention peuvent être également sous forme dite "auto-neutralisante" ou encore "auto-régulée" et dans ce cas, le composé de formule générale (I) est associé à au moins un disulfure soit connu pour son utilisation dans une composition réductrice pour permanente auto-neutralisante soit dérivant d'un composé de formule générale (I) ou de l'un de ses sels et correspondant à la formule générale (II) suivante :

-[S-CH₂-CH₂-NH-A-CO-NR₁R₂]₂ (II)

dans laquelle A, R₁ et R₂ ont les mêmes significations que celles données pour la formule générale (I).

Le disulfure peut également se présenter sous forme d'un sel cosmétiquement acceptable.

Parmi les disulfures connus, on peut notamment mentionner l'acide dithioglycolique, le dithioglycérol, la cystamine, la N,N'-diacétyl-cystamine, la cystine, la pantéthine, et les disulfures des N-(mercapto-alkyl)ω-hydroxyalkylamides décrits dans la demande de brevet européen EP 354 835, les disulfures des N-mono ou N,N-dialkylmercapto-4 butyramides décrits dans la demande de brevet EP 368 763, les disulfures des aminomercaptoalkylamides décrits dans la demande de brevet EP 403 267 et les disulfures des alkylaminomercaptoalkylamides décrits à la demande de brevet EP 432 000.

Parmi les disulfures de formule générale (II), on peut citer :
- le [dithiobis(éthylèneimino)]-2,2'bis acétamide,
- le [dithiobis(éthylèneimino)]-2,2'bis propionamide,
- le [dithiobis(éthylèneimino)]-3,3'bis propionamide,
- le [dithiobis(éthylèneimino)]-2,2'bis (méthylthio-4) butanamide,
- le [dithiobis(éthylèneimino)]-2,2'bis (méthyl-3) butanamide,
- le [dithiobis(éthylèneimino)]-2,2'bis (N-méthylacétamide),
- le [dithiobis(éthylèneimino)]-2,2'bis [N-(hydroxy-2-éthyl)acétamide],
- le [dithiobis(éthylèneimino)]-2,2'bis (N-méthylpropionamide),
- le [dithiobis(éthylèneimino)]-2,2'bis [N-(hydroxy-2-éthyl)propionamide], et
- le [dithiobis(éthylèneimino)]-3,3'bis [N-(hydroxy-2-éthyl)propionamide],
ainsi que leurs chlorhydrates.

Dans les compositions auto-neutralisantes, le disulfure est généralement présent en un rapport molaire de 0,5 à 2,5 et de préférence de 1 à 2 par rapport au composé de formule générale (I) ou de ses sels (voir brevet US 3.768.490).

La présente invention a en outre pour objet un procédé de déformation permanente des cheveux consistant, dans une première étape, à réduire les liaisons disulfures de la kératine par application, pendant environ 5 à 60 min, d'une composition réductrice telle que définie ci-dessus puis dans une seconde étape à reformer lesdites liaisons par application d'une composition oxydante ou éventuellement en laissant agir l'oxygène de l'air.

La présente invention a également pour objet un procédé d'ondulation des cheveux dans lequel on applique une composition réductrice telle que définie ci-dessus sur des cheveux mouillés préalablement enroulés sur des rouleaux ayant de 4 à 20 mm de diamètre, la composition pouvant éventuellement être appliquée au fur et à mesure de l'enroulage des cheveux ; on laisse ensuite agir la composition réductrice pendant un temps de 5 à 60 minutes, de préférence de 5 à 30 minutes, puis on rince abondamment après quoi on applique, sur les cheveux enroulés, une composition oxydante permettant de reformer les liaisons disulfures de la kératine pendant un temps de pose de 2 à 10 minutes. Après avoir enlevé les rouleaux, on rince abondamment la chevelure.

La composition d'oxydation ou oxydante est du type couramment utilisé et contient comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10. Cette oxydation peut être effectuée immédiatement ou être différée.

La présente invention a également pour objet un procédé de défrisage ou de décrêpage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60 minutes, en particulier de 5 à 30 minutes, on procède alors à un nouveau lissage puis on rince soigneusement et on applique une composition oxydante ou fixatrice telle que définie ci-dessus, que l'on laisse agir pendant environ 2 à 10 minutes puis on rince abondamment les cheveux.

La présente invention a également pour objet à titre de composés nouveaux les mercaptoalkylamino-amides répondant à la formule générale suivante :

HS-CH₂-CH₂-NH-A-CO-NR₁R₂ (III)

dans laquelle :
A, R₁ et R₂ ont les mêmes significations que données ci-dessus pour la formule générale (I) à l'exclusion des composés dans lesquels :
(i) A représente -(CH₂)₂- et R₁ et R₂ représentent un atome d'hydrogène, et
(ii) A représente 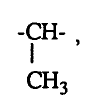 R₁ représente un atome d'hydrogène et R₂ représente un atome d'hydrogène ou le radical méthyle,
et les sels desdits composés de formule (III).

Parmi les mercaptoalkylamino-amides de formule générale (III), on peut notamment citer :
- le (mercapto-2 éthylamino)-2 acétamide,
- le (mercapto-2 éthylamino)-2 N-méthyl acétamide,
- le (mercapto-2 éthylamino)-2 N-éthyl acétamide,
- le (mercapto-2 éthylamino)-2 N-propyl acétamide,
- le (mercapto-2 éthylamino)-2 N-isopropyl acétamide,
- le (mercapto-2 éthylamino)-2 N,N-diméthyl acétamide,
- le (mercapto-2 éthylamino)-2 N,N-diéthyl acétamide,
- le (mercapto-2 éthylamino)-2 N-(hydroxy-2'éthyl) acétamide,
- le (mercapto-2 éthylamino)-2 N,N-di(hydroxy-2'éthyl) acétamide,
- le (mercapto-2 éthylamino)-3 N-méthyl propionamide,
- le (mercapto-2 éthylamino)-3 N-(hydroxy-2'éthyl)propionamide,
- le (mercapto-2 éthylamino)-2 N-(hydroxy-2'éthyl)propionamide,
- le (mercapto-2 éthylamino)-2 N,N-di(hydroxy-2'éthyl)propionamide,
- le (mercapto-2 éthylamino)-2 méthylthio-4 butanamide,
- le (mercapto-2 éthylamino)-2 méthylthio-4 N-méthylbutanamide,
- le (mercapto-2 éthylamino)-2 méthylthio-4 N-(hydroxy-2'éthyl)butanamide,
- le (mercapto-2 éthylamino)-2 méthyl-3 butanamide,
- le (mercapto-2 éthylamino)-2 méthyl-4 pentanamide, et
- le (mercapto-2 éthylamino)-2 méthyl-3 pentanamide,
ainsi que leurs chlorhydrates.

Les composés nouveaux selon l'invention de formule générale (III) sont préparés par action de l'ammoniac ou d'une amine primaire ou secondaire sur un mercaptoéthylamino-ester de formule (1) selon le schéma suivant : 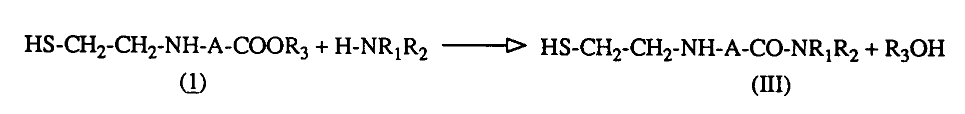
A, R₁ et R₂ ayant les mêmes significations que celles données pour la formule générale (I) et R₃ représente un reste alkyle en C₁-C₄ de préférence méthyle ou éthyle. La réaction est conduite sans solvant ou en présence d'un solvant de préférence polaire tel que par exemple l'eau, le méthanol, l'éthanol, l'isopropanol, le butanol-1, le pentanol-1, le diméthylformamide, le dichlorométhane, le dichloro-1,2 éthane, le trichloro-1,1,1 éthane, le chloroforme, l'acétonitrile, le dioxane, le tétrahydrofuranne ou le diméthoxy-1,2 éthane, à une température comprise entre la température ambiante et le point d'ébullition du solvant utilisé. Pour la préparation des amides primaires (R₁ = R₂ = H), on utilise de préférence une solution aqueuse d'ammoniaque mais on peut utiliser si nécessaire de l'ammoniaque gazeux. Il en est de même pour la mise en oeuvre des amines de bas point d'ébullition telles que par exemple la méthylamine ou l'éthylamine.

Les mercaptoéthylamino-esters de formule (1) sont obtenus par réaction d'un ester d'amino-acide (2) sur le sulfure d'éthylène, tel que décrit par exemple par L.G. Bulavin, Zh. Org. Khim., 7(10), 2086-2093 (1971), selon le schéma réactionnel suivant : 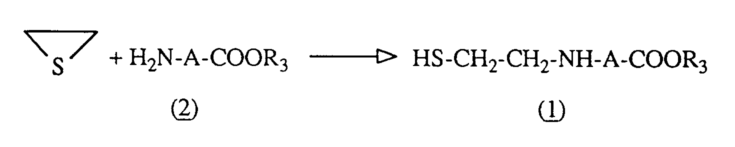
A et R₃ ont les mêmes significations que celles données ci-dessus. Les esters d'amino-acides de formule (2) sont préparés suivant les méthodes classiques connues.

La présente invention a également pour objet les nouveaux disulfures de formule générale (IV) suivante :

-[S-CH₂-CH₂-NH-A-CO-NR₁R₂]₂ (IV)

dans laquelle A, R₁ et R₂ ont les mêmes significations que celles données ci-dessus pour la formule générale (III).

Parmi les disulfures de formule générale (IV), on peut notamment citer :
- le [dithiobis(éthylèneimino)]-2,2'bis acétamide,
- le [dithiobis(éthylèneimino)]-2,2'bis (méthylthio-4 butanamide),
- le [dithiobis(éthylèneimino)]-2,2'bis (méthyl-3 butanamide),
- le [dithiobis(éthylèneimino)]-2,2'bis (N-méthylacétamide),
- le [dithiobis(éthylèneimino)]-2,2'bis [N-(hydroxy-2 éthyl)acétamide],
- le [dithiobis(éthylèneimino)]-2,2'bis [N-(hydroxy-2 éthyl)propionamide, et
- le [dithiobis(éthylèneimino)]-3,3'bis [N-(hydroxy-2 éthyl)propionamide],
ainsi que leurs chlorhydrates.

Ces disulfures sont obtenus par oxydation des composés de formule générale (III) soit à l'air soit en utilisant des oxydants connus comme par exemple l'eau oxygénée en présence éventuellement de sels métalliques tels que par exemple les sels ferreux.

On va maintenant donner à titre d'illustration et sans aucun caractère limitatif plusieurs exemples de préparation de composé selon l'invention ainsi que des exemples de compositions réductrices et leur utilisation dans un procédé de déformation permanente des cheveux.

### EXEMPLES DE PREPARATION

### EXEMPLE 1 : Préparation du chlorhydrate de (mercapto-2 éthylamino)-2 méthylthio-4 butanamide

### a) (Mercapto-2 éthylamino)-2 méthylthio-4 butanamide

Un mélange de 22,5 g (0,14 mole) de N-(mercapto-2 éthyl)méthioninate de méthyle et de 150 ml d'ammoniaque en solution aqueuse à 20 % est agité 4 heures à température ambiante. Après évaporation à sec sous pression réduite, on obtient 22 g du produit attendu sous forme d'une huile incolore. Le dosage de thiol est conforme au produit attendu.

### b) Chlorhydrate de (mercapto-2 éthylamino)-2 méthylthio-4 propionamide

Dans une solution de 22 g (0,1 mole)de (mercapto-2 éthyl amino)-2 méthylthio-4 propionamide et de 150 ml d'éthanol, refroidie à environ 5°C, on fait barboter de l'acide chlorhydrique gazeux pendant environ 1 heure. Après essorage du précipité et séchage sous vide à température ambiante, on obtient 17,4 g du chlorhydrate de (mercapto-2 éthylamino)-2 méthylthio-4 propionamide sous forme d'un solide blanc de point de fusion : 164-166°C.

Le spectre RMN¹H (500 MHz), le spectre RMN¹³C (100 MHz) et le dosage de thiol sont conformes à la structure du produit attendu.

### ANALYSE ELEMENTAIRE

| C₇H₁₆N₂OS₂, HCl | | | | | | |
|---|---|---|---|---|---|---|
| | C % | H % | N % | O % | S % | Cl % |
| Calculé | 34,34 | 7,00 | 11,44 | 6,54 | 26,20 | 14,48 |
| Trouvé | 34,18 | 7,11 | 11,34 | 7,16 | 25,98 | 14,28 |

### EXEMPLE 2 : Préparation du chlorhydrate de (mercapto-2 éthylamino)-2 acétamide

### a) (mercapto-2 éthylamino)-2 acétamide

Le mode opératoire est identique à celui décrit à l'exemple 1(a). A partir de 17 g (0,1 mode) de N-(mercapto-2 éthyl)glycinate d'éthyle et de 200 ml de solution aqueuse d'ammoniaque à 20 %, on obtient 13,4 g d'une huile incolore.

### b) Chlorhydrate de (mercapto-2 éthylamino)-2 acétamide

Le mode opératoire est identique à celui décrit à l'exemple 1(b). A partir de 13,4 g (0,1 mole) de (mercapto-2 éthylamino)-2 acétamide, on obtient 13,4 g d'un solide blanc hygroscopique.

Le spectre RMN¹H (80 MHz) et le dosage de thiol sont conformes à la structure du produit attendu.

### ANALYSE ELEMENTAIRE

| C₄H₁₀N₂OS, HCl | | | |
|---|---|---|---|
| | C % | H % | N % |
| Calculé | 28,15 | 6,50 | 16,41 |
| Trouvé | 28,07 | 6,49 | 15,87 |

### EXEMPLE 3 : Préparation du chlorhydrate de (mercapto-2 éthylamino)-2 propionamide

### a) (mercapto-2 éthylamino)-2 propionamide

Le mode opératoire est identique à celui décrit à l'exemple 1(a). A partir de 54 g (3,3.10⁻¹ modes) de N-(mercapto-2 éthyl)D,L-alaninate de méthyle et de 600 ml d'ammoniaque en solution aqueuse à 20 %, on obtient 48 g d'une huile jaune.

Le spectre RMN¹H (200 MHz) et le dosage de thiol sont conformes à la structure du produit attendu.

### b) Chlorhydrate de (mercapto-2 éthylamino)-2 propionamide

Le mode opératoire est identique à celui décrit à l'exemple 1(b). A partir de 48 g (3,2.10⁻¹ modes) de (mercapto-2 éthylamino)-2 propionamide et de 400 ml d'éthanol, on obtient 51 g du chlorhydrate de (mercapto-2 éthylamino)-2 propionamide sous la forme d'un solide jaune pâle de point de fusion 150-155°C.

Les spectres RMN¹H (500 MHz) et ¹³C (100 MHz) et le dosage de thiol sont conformes à la structure du produit attendu.

### ANALYSE ELEMENTAIRE

| C₅H₁₂N₂OS, HCl | | | | | | |
|---|---|---|---|---|---|---|
| | C % | H % | N % | O % | S % | Cl % |
| Calculé | 32,52 | 7,09 | 15,15 | 8,66 | 17,36 | 19,20 |
| Trouvé | 32,10 | 7,15 | 14,90 | 9,06 | 16,91 | 18,88 |

### EXEMPLE 4 : Préparation du chlorhydrate de (mercapto-2 éthylamino)-3 propionamide

### a) mercapto-2 éthylamino)-3 propionamide

Le mode opératoire est le même que pour l'exemple 1(a). A partir de 5,7 g (35 mmoles) de N-(mercapto-2 éthyl)β-alaninate de méthyle, on obtient 5,1 g du produit attendu sous la forme d'une huile incolore. Le dosage de thiol est conforme à la structure du produit attendu.

### b) Chlorhydrate de (mercapto-2 éthylamino)-3 propionamide

Le mode opératoire est le même que pour l'exemple 1(b). A partir de 5,1 g (34 mmoles) de (mercapto-2 éthylamino)-3 propionamide, on obtient 4,7 g du chlorhydrate de (mercapto-2 éthylamino)-3 propionamide sous la forme d'un solide blanc de point de fusion 116°C.

Le spectre RMN¹H (200 MHz) et le dosage de thiol sont conformes à la structure du produit attendu.

### ANALYSE ELEMENTAIRE

| C₅H₁₂N₂OS, HCl | | | | | | |
|---|---|---|---|---|---|---|
| | C % | H % | N % | O % | S % | Cl % |
| Calculé | 32,52 | 7,09 | 15,17 | 8,66 | 17,36 | 19,20 |
| Trouvé | 32,64 | 7,07 | 15,13 | 8,76 | 17,37 | 19,09 |

### EXEMPLE 5 : Préparation du chlorhydrate de (mercapto-2 éthylamino)-2 N-(hydroxy-2'éthyl)propionamide

### a) (mercapto-2 éthylamino)-2 N-(hydroxy-2' éthyl)propionamide

On chauffe à 65-70°C, sous agitation et sous argon, un mélange de 18 g (0,11 mode) de N-(mercapto-2 éthyl)D,L-alaninate de méthyle et de 6,7 g (0,11 mole) d'éthanolamine en distillant au fur et à mesure le méthanol formé. La réaction est complète en 4 heures. On refroidit à température ambiante, ajoute 100 cm³ d'eau et extrait 3 fois à l'éther éthylique. La phase aqueuse est évaporée à sec sous pression réduite. Après séchage prolongé sous vide à température ambiante, on obtient 15,6 g de (mercapto-2 éthylamino)-2 N-(hydroxy-2'éthyl)propionamide sous la forme d'un solide blanc pâteux.

Les spectres RMN¹H 200 MHz (DMSO d₆ et CDCl₃) ainsi que le dosage de thiol sont conformes à la structure du produit attendu.

### b) Chlorhydrate de (mercapto-2 éthylamino)-2 N-(hydroxy-2' éthyl)propionamide

On fait barboter de l'acide chlorhydrique gazeux dans une solution de 13 g (67 mmoles) du composé obtenu ci-dessus dans 100 cm³ d'éthanol, refroidit à 5°C, pendant environ 2 heures. Le précipité formé est essoré puis séché sous vide à température ambiante sur potasse. On obtient 11 g de chlorhydrate de (mercapto-2 éthylamino)-2 N-(hydroxy-2'éthyl)propionamide sous la forme d'un solide blanc de point de fusion : 121-123°C.

Le spectre RMN¹H 200 MHz (DMSO d₆) est conforme à la structure du produit attendu. Le dosage de thiol est également conforme

| | |
|---|---|
| trouvé | 4,31 meq/g |
| calculé | 4,37 meq/g |

### EXEMPLE 6 : Préparation du dichlorydrate du [dithiobis(éthylèneimino)]-2,2'bis propionamide

A une solution de 1,3 g (7 mmoles) de chlorhydrate de (mercapto-2 éthylamino)-2 propionamide obtenu à l'exemple 3, dans 20 cm³ d'un mélange éthanol/méthanol 50/50, on ajoute goutte à goutte 0,34 cm³ (3,5 mmoles) d'eau oxygénée à 110 volumes en maintenant la température inférieure à 25°C. La solution est abandonnée une nuit à température ambiante puis concentrée sous pression réduite jusqu'à début de cristallisation. On refroidit à 0°C, essore sur verre fritté, lave avec de l'éthanol absolu et sèche sous vide à 30°C. On obtient 1,05 g de dichlorhydrate du [dithiobis(éthylèneimino)]-2,2'bis propionamide sous la forme d'un solide blanc de point de fusion 210°C.

### ANALYSE ELEMENTAIRE

| C₁₀H₂₂N₄O₂S₂, 2HCl | | | | | | |
|---|---|---|---|---|---|---|
| | C % | H % | N % | O % | S % | Cl % |
| Calculé | 32,69 | 6,59 | 15,25 | 8,71 | 17,46 | 19,30 |
| Trouvé | 32,37 | 6,61 | 15,11 | 8,90 | 17,23 | 19,10 |

### EXEMPLES DE COMPOSITIONS

### EXEMPLE 1

### a) Composition réductrice

| | |
|---|---|
| - Chlorhydrate de (mercapto-2'éthylamino-2)propionamide | 18 g |
| - Cocoamidopropylbétaïne (Tegobétaïne HS® vendu par la Société Goldschmidt) | 0,2 g |
| - Sel pentasodique de l'acide diéthylène triamine pentacétique | 0,15 g |
| - Parfum qs | |
| - Ammoniaque qsp | pH 8 |
| - Eau déminéralisée qsp | 100 g |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 2

### a) Composition réductrice

| | |
|---|---|
| - Chlorhydrate de (mercapto-2 éthylamino)-2 propionamide | 10 g |
| - Oxyde de laurylamine (Aromox DMMCD/W® vendu par la Société AKZO) | 2 g |
| - Acide éthylène diamine tétracétique | 0,15 g |
| - Parfum qs | |
| - Monoéthanolamine qs | pH 8,5 |
| - Eau déminéralisée qsp | 100 g |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 3

### a) Composition réductrice

| | |
|---|---|
| - Chlorhydrate de (mercapto-2 éthylamino)-2 acétamide | 14 g |
| - Cocoamidopropylbétaïne (Tegobétaïne HS® vendu par la Société Goldschmidt) | 0,2 g |
| - Sel pentasodique de l'acide diéthylène triamine pentacétique | 0,2 g |
| - Parfum qs | |
| - Ammoniaque qsp | pH 8,5 |
| - Eau déminéralisée qsp | 100 g |

### b) Composition oxydante

| | |
|---|---|
| - Eau oxygénée | 2,0 g |
| - Chlorure d'oléocétyl diméthylhydroxyéthylammonium | 0,3 g |
| - Acide phosphorique | 0,5 g |
| - p-éthoxyacétonilide (phénacétine) | 0,1 g |
| - Hydrolysat de protéines | 0,5 g |
| - Parfum | 0,5 g |
| - Eau déminéralisée qsp | 100 g |

### EXEMPLE 4 :

### a) Composition réductrice

| | |
|---|---|
| - Chlorhydrate de (mercapto-2 éthylamino)-2 N-(hydroxy-2'éthyl)propionamide | 18 g |
| - Cocoamidopropylbétaïne vendu sous la dénomination de Tegobétaïne HS® par la Société Goldschmidt | 0,8 g |
| - Sel pentasodique de l'acide diéthylène triamine pentacétique | 0,15 g |
| - Parfum qs | |
| - Ammoniaque qsp | pH 8 |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 5 :

### a) Composition réductrice

| | |
|---|---|
| - Chlorhydrate de (mercapto-2 éthylamino)-2 méthylthio-4 butanamide | 16 g |
| - Oxyde de lauramine vendu sous la dénomination de Aromox DMMCD/W® par la Société AKZO | 2,1 g |
| - Acide éthylènediamine tétracétique | 0,2 g |
| - Parfum qs | |
| - Monoéthanolamine qsp | pH 8,2 |
| - Eau déminéralisée qsp | 100 g |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 6 :

### a) Composition réductrice

| | |
|---|---|
| - Chlorhydrate de (mercapto-2 éthylamino)-2 propionamide | 9 g |
| - Acide thioglycolique | 4,5 g |
| - Oxyde de laurylamine (Aromox DMMCD/W® vendu par la Société AKZO) | 1,2 g |
| - Homopolymère du chlorure de N,N-diméthyl N-2 propényl-2 propène-1 (polyquaternium-6) (Merquat 100® vendu par la Société Merck | 1 g |
| - Parfum qs | |
| - Ammoniaque (20 %) qs | pH 8,2 |
| - Eau déminéralisée qsp | 100 g |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 7 :

### a) Composition réductrice

| | |
|---|---|
| - Chlorhydrate de (mercapto-2 éthylamino)-2 propionamide | 16 g |
| - Cystéine | 4,5 g |
| - Ester stéarique polyéthyléné avec 8 moles d'oxyde d'éthyle (MYRJ 45® vendu par la Société ICI) | 0,85 g |
| - Conservateur | 0,35 g |
| - Parfum qs | |
| - Ammoniaque qs | pH 8,8 |
| - Eau déminéralisée qsp | 100 g |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 8 :

### a) Composition réductrice

| | |
|---|---|
| - Chlorhydrate de (mercapto-2 éthylamino)-2 propionamide | 10 g |
| - Chlorhydrate de cystéamine | 6 g |
| - Oxyde de laurylamine (Aromox DMMCK/W® vendu par la Société AKZO) | 2 g |
| - Conservateur | 0,15 g |
| - Parfum qs | |
| - Monoéthanolamine qs | pH 7,8 |
| - Eau déminéralisée qsp | 100 g |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 9 :

### a) Composition réductrice

| | |
|---|---|
| - Chlorhydrate de (mercapto-2 éthylamino)-3 propionamide | 7,2 g |
| - Thioglycolate de glycérol à 68 % de matière active (glycérol) | 5 g |
| - Chlorure d'oléocétyl diméthylhydroxyéthylammonium | 0,3 g |
| - Conservateur | 0,15 g |
| - Parfum qs | |
| - Ammoniaque qs | pH 7,2 |
| - Eau déminéralisée qsp | 100 g |

### b) Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

### EXEMPLE 10 :

### a) Composition réductrice

| | |
|---|---|
| - Chlorhydrate de (mercapto-2'éthylamino-2)propionamide | 18 g |
| - Cocoamidopropylbétaïne (Tegobétaïne HS® vendu par la Société Goldschmidt) | 0,2 g |
| - Sel pentasodique de l'acide diéthylène triamine pentacétique | 0,15 g |
| - Parfum qs | |
| - Ammoniaque qsp | pH 8 |
| - Eau déminéralisée qsp | 100 g |

### b) Composition oxydante

| | |
|---|---|
| - Bromate de sodium | 8 g |
| - Triéthanolamine qs | pH 8 |
| - Phosphate monosodique hydraté (12 H₂O) | 0,3 g |
| - Phosphate trisodique hydraté | 0,5 g |
| - Cocoamidopropylbétaïne (Tegobétaïne HS® vendu par la Société Goldschmidt) | 1 g |
| - Parfum qs | |
| - Eau déminéralisée qsp | 100 g |

## Revendications

1. Composition cosmétique pour le premier temps d'une opération de déformation permanente des cheveux, caractérisée par le fait qu'elle contient, dans un véhicule cosmétique approprié, en tant qu'agent réducteur, au moins un mercaptoalkylamino-amide ayant la formule générale suivante :
HS-CH₂-CH₂-NH-A-CO-NR₁R₂ (I)
dans laquelle :
A représente le radical divalent (̵CH₂)ₙ- , n étant un nombre entier compris entre 1 et 5, ou le radical 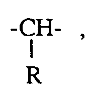 R représentant un radical alkyle inférieur, linéaire ou ramifié, ayant de 1 à 5 atomes de carbone ou le radical méthylthio-2 éthyle,
R₁ et R₂, identiques ou différents, représentent un atome d'hydrogène ou un radical alkyle inférieur, linéaire ou ramifié, ayant de 1 à 3 atomes de carbone ou un radical hydroxyalkyle ayant de 1 à 4 atomes de carbone,
ou un sel desdits composés de formule (I).

2. Composition selon la revendication 1, caractérisée par le fait que le sel du composé de formule (I) est choisi parmi les chlorhydrates, bromhydrates, citrates, oxalates et acétates.

3. Composition selon la revendication 1 ou 2, caractérisée par le fait que les composés de formule générale (I) sont choisis parmi :
- le (mercapto-2 éthylamino)-2 acétamide,
- le (mercapto-2 éthylamino)-2 propionamide,
- le (mercapto-2 éthylamino)-3 propionamide,
- le (mercapto-2 éthylamino)-2 méthylthio-4 butanamide,
- le (mercapto-2 éthylamino)-2 méthyl-3 butanamide,
- le (mercapto-2 éthylamino)-2 méthyl-4 pentanamide,
- le (mercapto-2 éthylamino)-2 méthyl-3 pentanamide,
- le (mercapto-2 éthylamino)-2 N-méthyl acétamide,
- le (mercapto-2 éthylamino)-2 N-éthyl acétamide,
- le (mercapto-2 éthylamino)-2 N-propyl acétamide,
- le (mercapto-2 éthylamino)-2 N-isopropyl acétamide,
- le (mercapto-2 éthylamino)-2 N,N-diméthyl acétamide,
- le (mercapto-2 éthylamino)-2 N,N-diéthyl acétamide,
- le (mercapto-2 éthylamino)-2 N-(hydroxy-2' éthyl)acétamide,
- le (mercapto-2 éthylamino)-2 N,N-di(hydroxy-2' éthyl)acétamide,
- le (mercapto-2 éthylamino)-2 N-méthyl propionamide,
- le (mercapto-2 éthylamino)-2 N,N-diméthyl propionamide,
- le (mercapto-2 éthylamino)-2 N-éthyl propionamide,
- le (mercapto-2 éthylamino)-2 N-(hydroxy-2' éthyl)propionamide,
- le (mercapto-2 éthylamino)-2 N,N-di(hydroxy-2' éthyl)propionamide,
- le (mercapto-2 éthylamino)-3 N-méthyl propionamide,
- le (mercapto-2 éthylamino)-3 N-(hydroxy-2' éthyl) propionamide,
- le (mercapto-2 éthylamino)-2 méthylthio-4 N-méthyl butanamide et
- le (mercapto-2 éthylamino)-2 méthylthio-4 N-(hydroxy-2' éthyl)butanamide,
ainsi que leurs chlorhydrates.

4. Composition selon l'une quelconque des revendications 1 à 3, caractérisée par le fait que le composé de formule (I) est présent à une concentration comprise entre 1 et 30 %, et de préférence entre 5 et 20 % en poids par rapport au poids total de la composition réductrice.

5. Composition selon l'une quelconque des revendications 1 à 4, caractérisée par le fait qu'elle présente un pH compris entre 4 et 11, et de préférence entre 6 et 10, obtenu à l'aide d'un agent alcalin choisi parmi l'ammoniaque, la monoéthanolamine, la diéthanolamine, la triéthanolamine, la propanediamine-1,3, un carbonate ou bicarbonate alcalin ou d'ammonium, un carbonate de guanidine, un hydroxyde alcalin ou à l'aide d'un agent acidifiant choisi parmi l'acide chlorhydrique, l'acide acétique, l'acide lactique, l'acide oxalique ou l'acide borique.

6. Composition selon l'une quelconque des revendications 1 à 5, caractérisée par le fait qu'elle contient en outre au moins un agent réducteur choisi parmi l'acide thioglycolique, le monothioglycolate de glycérol ou de glycol, la cystéamine, la N-acétylcystéamine, la N-propionylcystéamine, la cystéine, la N-acétylcystéine, le N-(mercapto-2 éthyl)gluconamide, l'acide β-mercaptopropionique et ses dérivés, l'acide thiolactique, le monothiolactate de glycérol, l'acide thiomalique, la panthétéine, le thioglycérol, un sulfite ou un bisulfite d'un métal alcalin ou alcalino-terreux, les N-(mercaptoalkyl)ω-hydroxyalkylamides et les N-mono ou N,N-dialkylmercapto-4-butyramides.

7. Composition selon l'une quelconque des revendications 1 à 6, caractérisée par le fait qu'elle contient en outre au moins un polymère cationique, un aminoacide basique ou acide, un peptide, un hydrolysat de protéines, une cire, un agent de gonflement et de pénétration, de l'acide panthothénique, un agent antichute, un agent antipelliculaire, un épaississant, un agent de suspension, un agent séquestrant, un agent opacifiant, un colorant, un filtre solaire, un parfum, un agent conservateur, un agent tensio-actif et un agent traitant.

8. Composition selon l'une quelconque des revendications 1 à 7, caractérisée par le fait qu'elle contient en outre au moins un disulfure, la composition étant du type auto-neutralisante.

9. Composition selon la revendication 8, caractérisée par le fait que le disulfure est l'acide dithioglycolique, le dithioglycérol, la cystamine, la N,N'-diacétyl-cystamine, la cystine, la panthétine ou un disulfure de N-(mercaptoalkyl)ω-hydroxyalkylamide ou de N-mono- ou N,N-dialkylmercapto-4-butyramide.

10. Composition selon la revendication 8, caractérisée par le fait que le disulfure correspond à la formule générale (II) suivante :
-[S-CH₂-CH₂-NH-A-CO-NR₁R₂]₂ (II)
dans laquelle A, R₁ et R₂ ont les mêmes significations que celles données pour la formule générale (I) de la revendication 1.

11. Composition selon la revendication 10, caractérisée par le fait que le disulfure de formule (II) est choisi parmi :
- le [dithiobis(éthylèneimino)]-2,2'bis acétamide,
- le [dithiobis(éthylèneimino)]-2,2'bis propionamide,
- le [dithiobis(éthylèneimino)]-3,3'bis propionamide,
- le [dithiobis(éthylèneimino)]-2,2'bis (méthylthio-4) butanamide,
- le [dithiobis(éthylèneimino)]-2,2'bis (méthyl-3) butanamide,
- le [dithiobis(éthylèneimino)]-2,2'bis (N-méthylacétamide),
- le [dithiobis(éthylèneimino)]-2,2'bis [N-(hydroxy-2-éthyl)acétamide],
- le [dithiobis(éthylèneimino)]-2,2'bis (N-méthylpropionamide),
- le [dithiobis(éthylèneimino)]-2,2'bis [N-(hydroxy-2-éthyl)propionamide], et
- le [dithiobis(éthylèneimino)]-3,3'bis [N-(hydroxy-2-éthyl)propionamide],
ainsi que leurs chlorhydrates.

12. Composition selon l'une quelconque des revendications 8 à 11, caractérisée par le fait que le disulfure est présent en une proportion molaire par rapport au composé de formule (I) allant de 0,5 à 2,5, et de préférence de 1 à 2.

13. Procédé de déformation permanente des cheveux consistant dans une première étape à réduire les liaisons disulfures de la kératine par application d'une composition réductrice, puis dans une seconde étape à reformer lesdites liaisons par application d'une composition oxydante, caractérisé par le fait que l'étape de réduction est réalisée à l'aide d'une composition cosmétique réductrice telle que revendiquée dans l'une quelconque des revendications 1 à 7.

14. Procédé selon la revendication 13, caractérisé par le fait que l'on laisse agir la composition réductrice pendant un temps compris entre 5 et 60 minutes.

15. Composés chimiques nouveaux, caractérisés par le fait qu'ils sont des mercaptoalkylamino-amides répondant à la formule générale suivante :
HS-CH₂-CH₂-NH-A-CO-NR₁R₂ (III)
dans laquelle :
A, R₁ et R₂ ont les mêmes significations que celles données à la revendication 1 pour la formule générale (I) à l'exclusion des composés dans lesquels :
(i) A représente -(CH₂)₂- et R₁ et R₂ représentent un atome d'hydrogène, et
(ii) A représente 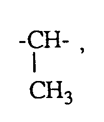 R₁ représente un atome d'hydrogène et R₂ représente un atome d'hydrogène ou le radical méthyle,
et les sels desdits composés de formule (III).

16. Composés selon la revendication 15, caractérisés par le fait qu'ils sont choisis parmi :
- le (mercapto-2 éthylamino)-2 acétamide,
- le (mercapto-2 éthylamino)-2 N-méthyl acétamide,
- le (mercapto-2 éthylamino)-2 N-éthyl acétamide,
- le (mercapto-2 éthylamino)-2 N-propyl acétamide,
- le (mercapto-2 éthylamino)-2 N-isopropyl acétamide,
- le (mercapto-2 éthylamino)-2 N,N-diméthyl acétamide,
- le (mercapto-2 éthylamino)-2 N,N-diéthyl acétamide,
- le (mercapto-2 éthylamino)-2 N-(hydroxy-2'éthyl) acétamide,
- le (mercapto-2 éthylamino)-2 N,N-di(hydroxy-2'éthyl) acétamide,
- le (mercapto-2 éthylamino)-3 N-méthyl propionamide,
- le (mercapto-2 éthylamino)-3 N-(hydroxy-2'éthyl)propionamide,
- le (mercapto-2 éthylamino)-2 N-(hydroxy-2'éthyl)propionamide,
- le (mercapto-2 éthylamino)-2 N,N-di(hydroxy-2'éthyl)propionamide,
- le (mercapto-2 éthylamino)-2 méthylthio-4 butanamide,
- le (mercapto-2 éthylamino)-2 méthylthio-4 N-méthylbutanamide,
- le (mercapto-2 éthylamino)-2 méthylthio-4 N-(hydroxy-2'éthyl)butanamide,
- le (mercapto-2 éthylamino)-2 méthyl-3 butanamide,
- le (mercapto-2 éthylamino)-2 méthyl-4 pentanamide, et
- le (mercapto-2 éthylamino)-2 méthyl-3 pentanamide,
ainsi que leurs chlorhydrates.

17. Procédé de préparation des mercaptoalkylamino-amides de formule (III) selon les revendications 15 et 16, caractérisé par le fait qu'il consiste à faire réagir, avec ou sans solvant, de l'ammoniac ou une amine primaire ou secondaire de formule H-NR₁R₂ sur un mercaptoéthylamino-ester de formule :
HS-CH₂-CH₂-NH-A-COOR₃
A, R₁ et R₂ ayant les mêmes significations que celles données à la revendication 15 pour la formule générale (III) et R₃ représente un reste alkyle en C₁-C₄.

18. Procédé selon la revendication 17, caractérisé par le fait que la réaction est réalisée en présence d'un solvant polaire choisi parmi l'eau, le méthanol, l'éthanol, l'isopropanol, le butanol-1, le pentanol-1, le diméthylformamide, le dichlorométhane, le dichloro-1,2 éthane, le trichloro-1,1,1-éthane, le chloroforme, l'acétonitrile, le dioxane, le tétrahydrofuranne ou le diméthoxy-1,2 éthane.

19. Composés nouveaux caractérisés par le fait qu'ils répondent à la formule générale suivante :
-[S-CH₂-CH₂-NH-A-CO-NR₁R₂]₂ (IV)
dans laquelle A, R₁ et R₂ ont les mêmes significations que celles données à la revendication 15 pour la formule générale (III).

20. Composés selon la revendication 19, caractérisés par le fait qu'ils sont choisis parmi :
- le [dithiobis(éthylèneimino)]-2,2'bis acétamide,
- le [dithiobis(éthylèneimino)]-2,2'bis (méthylthio-4 butanamide),
- le [dithiobis(éthylèneimino)]-2,2'bis (méthyl-3 butanamide),
- le [dithiobis(éthylèneimino)]-2,2'bis (N-méthylacétamide),
- le [dithiobis(éthylèneimino)]-2,2'bis [N-(hydroxy-2 éthyl)acétamide],
- le [dithiobis(éthylèneimino)]-2,2'bis [N-(hydroxy-2 éthyl)propionamide, et
- le [dithiobis(éthylèneimino)]-3,3'bis [N-(hydroxy-2 éthyl)propionamide],
ainsi que leurs chlorhydrates.

## Claims

1. Cosmetic composition for the first stage of an operation for the permanent deformation of the hair, characterized in that it contains, in an appropriate cosmetic vehicle, as reducing agent, at least one (mercaptoalkylamino)amide having the following general formula:
HS-CH₂-CH₂-NH-A-CO-NR₁R₂ (I)
in which:
A represents the divalent radical (̵CH₂)ₙ- , n being an integer between 1 and 5,
or the radical 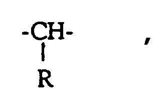 R representing a linear or branched lower alkyl radical having from 1 to 5 carbon atoms or the 2-methylthioethyl radical,
R₁ and R₂, which are identical or different, represent a hydrogen atom or a linear or branched lower alkyl radical having from 1 to 3 carbon atoms or a hydroxyalkyl radical having from 1 to 4 carbon atoms,
or a salt of the said compounds of formula (I).

2. Composition according to Claim 1, characterized in that the salt of the compound of formula (I) is chosen from the hydrochlorides, hydrobromides, citrates, oxalates and acetates.

3. Composition according to Claim 1 or 2, characterized in that the compounds of general formula (I) are chosen from:
- 2-(2-mercaptoethylamino)acetamide,
- 2-(2-mercaptoethylamino)propionamide,
- 3-(2-mercaptoethylamino)propionamide,
- 2-(2-mercaptoethylamino)-4-methylthiobutanamide,
- 2-(2-mercaptoethylamino)-3-methylbutanamide,
- 2-(2-mercaptoethylamino)-4-methylpentanamide,
- 2-(2-mercaptoethylamino)-3-methylpentanamide,
- 2-(2-mercaptoethylamino)-N-methylacetamide,
- 2-(2-mercaptoethylamino)-N-ethylacetamide,
- 2-(2-mercaptoethylamino)-N-propylacetamide,
- 2-(2-mercaptoethylamino)-N-isopropylacetamide,
- 2-(2-mercaptoethylamino)-N,N-dimethylacetamide,
- 2-(2-mercaptoethylamino)-N,N-diethylacetamide,
- 2-(2-mercaptoethylamino)-N-(2'-hydroxyethyl)acetamide,
- 2-(2-mercaptoethylamino)-N,N-di(2'-hydroxyethyl)acetamide,
- 2-(2-mercaptoethylamino)-N-methylpropionamide,
- 2-(2-mercaptoethylamino)-N,N-dimethylpropionamide,
- 2-(2-mercaptoethylamino)-N-ethylpropionamide,
- 2-(2-mercaptoethylamino)-N-(2'-hydroxyethyl)propionamide,
- 2-(2-mercaptoethylamino)-N,N-di(2'-hydroxyethyl)propionamide,
- 3-(2-mercaptoethylamino)-N-methylpropionamide,
- 3-(2-mercaptoethylamino)-N-(2'-hydroxyethyl)propionamide,
- 2-(2-mercaptoethylamino)-4-methylthio-N-methylbutanamide and,
- 2-(2-mercaptoethylamino)-4-methylthio-N-(2'-hydroxyethyl)butanamide,
as well as their hydrochlorides.

4. Composition according to any one of Claims 1 to 3, characterized in that the compound of formula (I) is present at a concentration of between 1 and 30%, and preferably between 5 and 20% by weight relative to the total weight of the reducing composition.

5. Composition according to any one of Claims 1 to 4, characterized in that it has a pH of between 4 and 11, preferably of between 6 and 10, obtained by means of an alkaline agent chosen from ammonium hydroxide, monoethanolamine, diethanolamine, triethanolamine, 1,3-propanediamine, an alkali metal or ammonium carbonate or bicarbonate, a guanidine carbonate, an alkali metal hydroxide, or by means of an acidifying agent chosen from hydrochloric acid, acetic acid, lactic acid, oxalic acid or boric acid.

6. Composition according to any one of Claims 1 to 5, characterized in that it contains, in addition, at least one reducing agent chosen from thioglycolic acid, glycerol or glycol monothioglycolate, cysteamine, N-acetylcysteamine, N-propionylcysteamine, cysteine, N-acetylcysteine, N-(2-mercaptoethyl)gluconamide, β-mercaptopropionic acid and its derivatives, thiolactic acid, glycerol monothiolactate, thiomalic acid, pantetheine, thioglycerol, a sulphite or bisulphite of an alkali or alkaline-earth metal, N-(mercaptoalkyl)ω-hydroxyalkylamides and N-mono- or N,N-dialkyl-4-mercaptobutyramides.

7. Composition according to any one of Claims 1 to 6, characterized in that it contains, in addition, at least one cationic polymer, a basic or acidic amino acid, a peptide, a protein hydrolysate, a wax, a swelling and penetrating agent, pantothenic acid, an agent for preventing hair loss, an antidandruff agent, a thickener, a suspending agent, a sequestering agent, an opacifying agent, a colorant, a sunscreen, a perfume, a preservative, a surfactant and a treatment agent.

8. Composition according to any one of Claims 1 to 7, characterized in that it contains, in addition, at least one disulphide, the composition being of the self-neutralizing type.

9. Composition according to Claim 8, characterized in that the disulphide is dithioglycolic acid, dithioglycerol, cystamine, N,N'-diacetylcystamine, cystine, pantethine or an N-(mercaptoalkyl)ω-hydroxyalkylamide or N-mono- or N,N-dialkyl-4-mercaptobutyramide disulphide.

10. Composition according to Claim 8, characterized in that the disulphide corresponds to the following general formula (II):
-[S-CH₂-CH₂-NH-A-CO-NR₁R₂]₂ (II)
in which A, R₁ and R₂ have the same meanings as those given for the general formula (I) of Claim 1.

11. Composition according to Claim 10, characterized in that the disulphide of formula (II) is chosen from:
- 2,2'-[dithiobis(ethyleneimino)]bisacetamide,
- 2,2'-[dithiobis(ethyleneimino)]bispropionamide,
- 3,3'-[dithiobis(ethyleneimino)]bispropionamide,
- 2,2'-[dithiobis(ethyleneimino)]bis(4-methylthiobutanamide),
- 2,2'-[dithiobis(ethyleneimino)]bis(3-methylbutanamide),
- 2,2'-[dithiobis(ethyleneimino)]bis(N-methylacetamide),
- 2,2'-[dithiobis(ethyleneimino)]bis[N-(2-hydroxyethyl)acetamide],
- 2,2'-[dithiobis(ethyleneimino)]bis(N-methylpropionamide),
- 2,2'-[dithiobis(ethyleneimino)]bis[N-(2-hydroxyethyl)propionamide], and
- 3,3'-[dithiobis(ethyleneimino)]bis[N-(2-hydroxyethyl)propionamide],
as well as their hydrochlorides.

12. Composition according to any one of Claims 8 to 11, characterized in that the disulphide is present in a molar proportion relative to the compound of formula (I) ranging from 0.5 to 2.5, and preferably from 1 to 2.

13. Process for the permanent deformation of the hair consisting, in a first stage, in reducing the disulphide bonds of keratin by application of a reducing compound, and then, in a second stage, in reforming the said bonds by application of an oxidizing composition, characterized in that the reducing stage is performed by means of a reducing cosmetic composition as claimed in any one of Claims 1 to 7.

14. Process according to Claim 13, characterized in that the reducing composition is allowed to react for a period of between 5 and 60 minutes.

15. New chemical compounds, characterized in that they are (mercaptoalkylamino)amides corresponding to the following general formula:
HS-CH₂-CH₂-NH-A-CO-NR₁R₂ (III)
in which:
A, R₁ and R₂ have the same meanings as those given in Claim 1 for the general formula (I) with the exception of the compounds in which:
(i) A represents -(CH₂)₂- and R₁ and R₂ represent a hydrogen atom, and
(ii) A represents 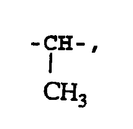 R₁ represents a hydrogen atom and R₂ represents a hydrogen atom or the methyl radical,
and the salts of the said compounds of formula (III).

16. Compounds according to Claim 15, characterized in that they are chosen from:
- 2-(2-mercaptoethylamino)acetamide,
- 2-(2-mercaptoethylamino)-N-methylacetamide,
- 2-(2-mercaptoethylamino)-N-ethylacetamide,
- 2-(2-mercaptoethylamino)-N-propylacetamide,
- 2-(2-mercaptoethylamino)-N-isopropylacetamide,
- 2-(2-mercaptoethylamino)-N,N-dimethylacetamide,
- 2-(2-mercaptoethylamino)-N,N-diethylacetamide,
- 2-(2-mercaptoethylamino)-N-(2'-hydroxyethyl)acetamide,
- 2-(2-mercaptoethylamino)-N,N-di(2'-hydroxyethyl)acetamide,
- 3-(2-mercaptoethylamino)-N-methylpropionamide,
- 3-(2-mercaptoethylamino)-N-(2'-hydroxyethyl)propionamide,
- 2-(2-mercaptoethylamino)-N-(2'-hydroxyethyl)propionamide,
- 2-(2-mercaptoethylamino)-N,N-di(2'-hydroxyethyl)propionamide,
- 2-(2-mercaptoethylamino)-4-methylthiobutanamide,
- 2-(2-mercaptoethylamino)-4-methylthio-N-methylbutanamide,
- 2-(2-mercaptoethylamino)-4-methylthio-N-(2'-hydroxyethyl)butanamide,
- 2-(2-mercaptoethylamino)-3-methylbutanamide,
- 2-(2-mercaptoethylamino)-4-methylpentanamide and,
- 2-(2-mercaptoethylamino)-3-methylpentanamide,
as well as their hydrochlorides.

17. Process for the preparation of the (mercaptoalkylamino)amides of formula (III) according to Claims 15 and 16, characterized in that it consists in reacting, with or without solvent, ammonia or a primary or secondary amine of formula H-NR₁R₂ with a mercaptoethylamino ester of formula:
HS-CH₂-CH₂-NH-A-COOR₃
A, R₁ and R₂ having the same meanings as those given in Claim 15 for the general formula (III) and R₃ represents a C₁-C₄ alkyl residue.

18. Process according to Claim 17, characterized in that the reaction is performed in the presence of a polar solvent chosen from water, methanol, ethanol, isopropanol, 1-butanol, 1-pentanol, dimethylformamide, dichloromethane, 1,2-dichloroethane, 1,1,1-trichloroethane, chloroform, acetonitrile, dioxane, tetrahydrofuran or 1,2-dimethoxyethane.

19. New compounds characterized in that they correspond to the following general formula:
-[S-CH₂-CH₂-NH-A-CO-NR₁R₂]₂ (IV)
in which A, R₁ and R₂ have the same meanings as those given in Claim 15 for the general formula (III).

20. Compounds according to Claim 19, characterized in that they are chosen from:
- 2,2'-[dithiobis(ethyleneimino)]bisacetamide,
- 2,2'-[dithiobis(ethyleneimino)]bis(4-methylthiobutanamide),
- 2,2'-[dithiobis(ethyleneimino)]bis(3-methylbutanamide),
- 2,2'-[dithiobis(ethyleneimino)]bis(N-methylacetamide),
- 2,2'-[dithiobis(ethyleneimino)]bis[N-(2-hydroxyethyl)acetamide],
- 2,2'-[dithiobis(ethyleneimino)]bis[N-(2-hydroxyethyl)propionamide], and
- 3,3'-[dithiobis(ethyleneimino)]bis[N-(2-hydroxyethyl)propionamide],
as well as their hydrochlorides.

## Patentansprüche

1. Kosmetische Zubereitung zur erstmaligen Anwendung eines Dauerwellverfahrens der Haare, die dadurch gekennzeichnet ist, daß sie in einem geeigneten kosmetischen Träger als Reduktionsmittel mindestens ein Mercaptoalkylaminoamid der folgenden allgemeinen Formel (I) enthält:
HS-CH₂-CH₂-NH-A-CO-NR₁R₂ (I)
in der
A einen divalenten Rest -(-CH₂-)ₙ-, wobei n eine ganze Zahl zwischen 1 und 5 ist, oder den Rest -CRH- darstellt, wobei R ein linearer oder verzweigter niedriger Alkylrest mit 1 bis 5 Kohlenstoffatomen oder ein 2-Methylthioethylrest ist, und
R₁ und R₂, die gleich oder verschieden sind, ein Wasserstoffatom, einen linearen oder verzweigten niedrigen Alkylrest mit 1 bis 3 Kohlenstoffatomen oder einen Hydroxyalkylrest mit 1 bis 4 Kohlenstoffatomen darstellen,
oder ein Salz der Verbindungen der Formel (I).

2. Zubereitung gemäß Anspruch 1, dadurch gekennzeichnet, daß das Salz der Verbindungen der Formel (I) unter deren Hydrochloriden, Hydrobromiden, Citraten, Oxalaten und Acetaten ausgewählt ist.

3. Zubereitung gemäß Anspruch 1 oder 2, dadurch gekennzeichnet, daß die Verbindungen der allgemeinen Formel (I) ausgewählt sind aus:
- 2-(2-Mercaptoethylamino)-acetamid,
- 2-(2-Mercaptoethylamino)-propionamid,
- 3-(2-Mercaptoethylamino)-propionamid,
- 2-(2-Mercaptoethylamino)-4-methylthiobutyramid,
- 2-(2-Mercaptoethylamino)-3-methylbutyramid,
- 2-(2-Mercaptoethylamino)-4-methylvaleramid,
- 2-(2-Mercaptoethylamino)-3-methylvaleramid,
- 2-(2-Mercaptoethylamino)-N-methylacetamid,
- 2-(2-Mercaptoethylamino)-N-ethylacetamid,
- 2-(2-Mercaptoethylamino)-N-propylacetamid,
- 2-(2-Mercaptoethylamino)-N-isopropylacetamid,
- 2-(2-Mercaptoethylamino)-N,N-dimethylacetamid,
- 2-(2-Mercaptoethylamino)-N,N-diethylacetamid,
- 2-(2-Mercaptoethylamino)-N-(2'-hydroxyethyl)-acetamid,
- 2-(2-Mercaptoethylamino)-N,N-di-(2'-hydroxyethyl)-acetamid,
- 2-(2-Mercaptoethylamino)-N-methylpropionamid,
- 2-(2-Mercaptoethylamino)-N,N-dimethylpropionamid,
- 2-(2-Mercaptoethylamino)-N-ethylpropionamid,
- 2-(2-Mercaptoethylamino)-N-(2'-hydroxyethyl)-propionamid,
- 2-(2-Mercaptoethylamino)-N,N-di-(2'-hydroxyethyl)-propionamid,
- 3-(2-Mercaptoethylamino)-N-methylpropionamid,
- 3-(2-Mercaptoethylamino)-N-(2'-hydroxyethyl)-propionamid,
- 2-(2-Mercaptoethylamino)-4-methylthio-N-methylbutyramid und
- 2-(2-Mercaptoethylamino)-4-methylthio-N-(2-hydroxyethyl)-butyramid
oder deren Hydrochloriden.

4. Zubereitung gemäß einem der Ansprüche 1 bis 3, dadurch gekennzeichnet, daß die Verbindung der Formel (I) in einer Konzentration zwischen 1 und 30 Gew.-% und vorzugsweise zwischen 5 und 20 Gew.-%, bezogen auf das Gesamtgewicht der reduzierenden Zubereitung, vorhanden ist.

5. Zubereitung gemäß einem der Ansprüche 1 bis 4, dadurch gekennzeichnet, daß sie einen pH zwischen 4 und 11 und vorzugsweise zwischen 6 und 10 aufweist, der mit Hilfe eines aus Ammoniak, Monoethanolamin, Diethanolamin, Triethanolamin, 1,3-Propandiamin, Alkali- oder Ammoniumcarbonat oder -bicarbonat, Guanidincarbonat und einem Alkalihydroxid ausgewählten alkalischen Mittels oder eines aus Salzsäure, Essigsäure, Milchsäure, Oxalsäure oder Borsäure ausgewählten Säuerungsmittels eingestellt wird.

6. Zubereitung gemäß einem der Ansprüche 1 bis 5, dadurch gekennzeichnet, daß sie zusätzlich mindestens ein Reduktionsmittel enthält, das aus Thioglykolsäure, Glykoloder Glycerinmonothioglykolat, Cysteamin, N-Acetylcysteamin, N-Propionylcysteamin, Cystein, N-Acerylcystein, N-(2-Mercaptoethyl)-gluconamid, der β-Mercaptopropionsäure und deren Derivaten, Thiomilchsäure, Glycerinmonothiolactat, Thiomaleinsäure, Panthetein, Thioglycerin, einem Alkali- oder Erdalkalimetallsulfit oder -bisulfit, N-(Mercaptoalkyl)-ω-hydroxyalkylamiden sowie N-Mono- oder N,N-Dialkyl-4-mercaptobutyramiden ausgewählt ist.

7. Zubereitung gemäß einem der Ansprüche 1 bis 6, dadurch gekennzeichnet, daß sie zusätzlich mindestens ein kationisches Polymer, eine basische oder saure Aminosäure, ein Peptid, ein Proteinhydrolysat, ein Wachs, ein Treibmittel oder Penetrationsmittel, Panthothensäure, Mittel gegen Haarausfall oder Schuppen, ein Verdickungsmittel, ein Suspendiermittel, einen Komplexbildner, ein Trübungsmittel, einen Farbstoff, einen UV-Filter, ein Parfum, ein Konservierungsmittel, ein oberflächenaktives Mittel und ein Behandlungsmittel enthält.

8. Zubereitung gemaß einem der Ansprüche 1 bis 7, dadurch gekennzeichnet, daß sie zusätzlich mindestens ein Disulfit enthält, wobei die Zubereitung selbstneutralisierenden ist.

9. Zubereitung gemäß Anspruch 8, dadurch gekennzeichnet, daß es sich bei dem Disulfit um Dithioglykolsäure, Dithioglycerin, Cystamin, N,N-Diacerylcystamin, Cystin, Panthetin, oder ein Disulfid eines N-(Mercaptoalkyl)-ω-hydroxyalkylamids sowie eines N-Mono- oder N,N-Dialkyl-4-mercaptobutyramids handelt.

10. Zubereitung gemäß Anspruch 8, dadurch gekennzeichnet, daß das Disulfit der folgenden allgemeinen Formel (II) entspricht:
-[S-CH₂-CH₂-NH-A-CO-NR₁R₂]₂ (II)
in der A, R₁ und R₂ dieselbe Bedeutung haben, die für die allgemeine Formel (I) in Anspruch 1 angegeben ist.

11. Zubereitung gemäß Anspruch 10, dadurch gekennzeichnet, daß das Disulfid der Formel (II) ausgewählt ist aus:
- 2,2'-[Dithiobis(ethylenimino)]-bisacetamid,
- 2,2'-[Dithiobis(ethylenimino)]-bispropionamid,
- 3,3'-[Dithiobis(ethylenimino)]-bispropionamid,
- 2,2'-[Dithiobis(ethylenimino)]-bis(4-methylthio)-butyramid,
- 2,2'-[Dithiobis(ethylenimino)]-bis(3-methyl)-butyramid,
- 2,2'-[Dithiobis(ethylenimino)]-bis(N-methylacetamid),
- 2,2'-[Dithiobis(ethylenimino)]-bis[N-(2-hydroxyethyl)acetamid],
- 2,2'-[Dithiobis(ethylenimino)]-bis(N-methylpropionamid),
- 2,2'-[Dithiobis(ethylenimino)]-bis[N-(2-hydroxyethyl)-propionamid] und
- 3,3'-[Dithiobis(ethylenimino)]-bis[N-(2-hydroxyethyl)-propionamid]
sowie deren Hydrochloriden

12. Zubereitung gemäß einem der Ansprüche 8 bis 11, dadurch gekennzeichnet, daß das Disulfid bezogen auf die Verbindung der Formel (I) in einem molaren Anteil von 0,5 bis 2,5 und vorzugsweise von 1 bis 2 vorliegt.

13. Dauerwellverfahren für Haare, bei dem man in einem ersten Schritt die Disulfidbindungen des Keratins durch Auftragen einer reduzierenden Zubereitung reduziert und dann in einem zweiten Schritt diese Bindungen durch Auftragen einer oxidierenden Zubereitung zurückbildet, wobei das Verfahren dadurch gekennzeichnet ist, daß der Reduktionsschritt mit Hilfe einer reduzierenden kosmetischen Zubereitung gemäß einem der Ansprüche 1 bis 7 durchgeführt wird.

14. Verfahren gemäß Anspruch 13, dadurch gekennzeichnet, daß man die reduzierende Zubereitung für ein Zeitspanne von 5 bis 60 Minuten einwirken läßt.

15. Neue chemische Verbindungen, dadurch gekennzeichnet, daß es sich um der folgenden allgemeinen Formel (III) entsprechende Mercaptoalkylaminoamide handelt:
HS-CH₂-CH₂-NH-A-CO-NR₁R₂ (III)
in der
A, R₁ und R₂die in Anspruch 1 für die allgemeine Formel (I) gegebene Bedeutung haben, mit Ausnahme derjenigen Verbindungen, in denen
(i) A den Rest -(CH₂)₂- darstellt und R₁ und R₂ Wasserstoffatome sind und
(ii) A den Rest -CH(CH₃)- darstellt, R₁ ein Wasserstoffatom und R₂ ein Wasserstoffatom oder ein Methylrest ist.
sowie Salze der Verbindungen der Formel (III).

16. Verbindungen gemäß Anspruch 15, dadurch gekennzeichnet, daß sie ausgewählt sind aus:
- 2-(2-Mercaptoethylamino)-acetamid,
- 2-(2-Mercaptoethylamino)-N-methylacetamid,
- 2-(2-Mercaptoethylamino)-N-ethylacetamid,
- 2-(2-Mercaptoethylamino)-N-propylacetamid,
- 2-(2-Mercaptoethylamino)-N-isopropylacetamid,
- 2-(2-Mercaptoethylamino)-N,N-dimethylacetamid,
- 2-(2-Mercaptoethylamino)-N,N-diethylacetamid,
- 2-(2-Mercaptoethylamino)-N-(2'-hydroxyethyl)-acetamid,
- 2-(2-Mercaptoethylamino)-N,N-di-(2'-hydroxyethyl)-acetamid,
- 3-(2-Mercaptoethylamino)-N-methylpropionamid,
- 3-(2-Mercaptoethylamino)-N-(2'-hydroxyethyl)-propionamid,
- 2-(2-Mercaptoethylamino)-N-(2'-hydroxyethyl)-propionamid,
- 2-(2-Mercaptoethylamino)-N,N-di-(2'-hydroxyethyl)-propionamid,
- 2-(2-Mercaptoethylamino)-4-methylthiobutyramid,
- 2-(2-Mercaptoethylamino)-4-methylthio-N-methylbutyramid,
- 2-(2-Mercaptoethylamino)-4-methylthio-N-(2'-hydroxyethyl)-butyramid,
- 2-(2-Mercaptoethylamino)-3-methylbutyramid,
- 2-(2-Mercaptoethylamino)-4-methylvaleramid und
- 2-(2-Mercaptoethylamino)-3-methylvaleramid
sowie deren Hydrochloriden.

17. Verfahren zur Herstellung der Mercaptoalkylaminoamide der Formel (III) gemäß dem Ansprüchen 15 und 16, dadurch gekennzeichnet, daß man Ammoniak, ein primäres oder ein sekundäres Amin der Formel H-NR₁R₂ mit oder ohne Losungsmittel mit einem Mercaptoethylaminoester der Formel:
HS-CH₂-CH₂-NH-A-COOR₃
reagieren läßt, in der
A, R₁ und R₂ die in Anspruch 15 für die allgemeine Formel (III) angegebene Bedeutung haben und R₃ einen C₁-C₄-Alkylrest darstellt.

18. Verfahren gemäß Anspruch 17, dadurch gekennzeichnet, daß die Reaktion in Anwesenheit eines polaren Lösungsmittels durchgeführt wird, welches aus Methanol, Ethanol, Isopropanol, 1-Butanol, 1-Pentanol, Dimethylformamid, Dichlormethan, 1,2-Dichlorethan, 1,1,1-Trichlorethan, Chloroform, Acetonitril, Dioxan, Tetrahydrofuran oder 1,2-Dimethoxyethan ausgewählt ist.

19. Neue Verbindungen, dadurch gekennzeichnet, daß sie der folgenden allgemeinen Formel (IV) entsprechen:
-[S-CH₂-CH₂-NH-A-CO-NR₁R₂]₂ (IV)
in der
A, R₁ und R₂ die in Anspruch 15 für die allgemeine Formel (III) angegebene Bedeutung haben.

20. Verbindungen gemäß Anspruch 19, dadurch gekennzeichnet, daß sie ausgewählt sind aus:
- 2,2'-[Dithiobis(ethylenimino)]-bisacetamid,
- 2,2'-[Dithiobis(ethylenimino)]-bis(4-methylthio)-butyramid,
- 2,2'-[Dithiobis(ethylenimino)]-bis(3-methyl)-butyramid,
- 2,2'-[Dithiobis(ethylenimino)]-bis(N-methylacetamid),
- 2,2'-[Dithiobis(ethylenimino)]-bis[N-(2'-hydroxyethyl)-acetamid],
- 2,2'-[Dithiobis(ethylenimino)]-bis[N-(2'-hydroxyethyl)-propionamid] und
- 3,3'-[Dithiobis(ethylenimino)]-bis[N-(2-hydroxyethyl)-propionamid]
sowie deren Hydrochloriden.
